**Europäisches Patentamt**

(19) **European Patent Office**

**Office européen des brevets**

(11) Publication number: **0 128 042**
**A2**

(12) **EUROPEAN PATENT APPLICATION**

(21) Application number: **84303784.7**

(22) Date of filing: **05.06.84**

(51) Int. Cl.³: **C 12 Q 1/68**
**C 12 N 15/00**

(30) Priority: **06.06.83 US 501351**
**20.06.83 US 506077**

(43) Date of publication of application:
**12.12.84 Bulletin 84/50**

(84) Designated Contracting States:
**AT BE CH DE FR GB IT LI LU NL SE**

(71) Applicant: **GENENTECH, INC.**
**460 Point San Bruno Boulevard**
**South San Francisco California 94080(US)**

(72) Inventor: **Berman, Cara Helene**
**971 Page Street**
**San Francisco California 94117(US)**

(72) Inventor: **Ullrich, Axel**
**433 Upper Terrace**
**San Francisco California 94117(US)**

(74) Representative: **Armitage, Ian Michael et al,**
**MEWBURN ELLIS & CO. 2/3 Cursitor Street**
**London EC4A 1BQ(GB)**

(54) A process for indentifying or isolating a DNA sequence, and a DNA hybridization probe therefor.

(57) A process for isolating a desired DNA sequence from a gene bank using a synthetic DNA hybridization probe of at least about 30 nucleotide base pairs. The probe is synthesized without regard for the degeneracy of the genetic code as it has been discovered that sufficiently long probe will hybridize under controlled conditions to the desired DNA even if not completely homologous with its complementary DNA with sufficient specificity to permit identification and isolation of the desired DNA.

HOMOLOGY BETWEEN IGF-1 AND SYNTHETIC IGF-1

```
IGF-1            AGACGCTCTG CGGGGCTGAG CTGGTGGATG CTCTTCAGTT CTGGTGTGGA GACAGGGGCT
                 * ** ** ** *** ***** ***** ** * **** **** *   ***** *** * ****
Synthetic IGF-1  AAACTCTGTG CGGTGCTGAA CTGGTTGACG CTCTGGAGTT CGTATGTGGT GACCGTGGCT


IGF-1            TTTATTTCAG TAAG
                 **** **** **
Synthetic IGF-1  TTTACTTCAA CAAA
```

Fig.1.

EP 0 128 042 A2

Docket 100/130,174

−1−

## A PROCESS FOR IDENTIFYING OR ISOLATING A DNA SEQUENCE, AND A DNA HYBRIDIZATION PROBE THEREFOR

### Field of the Invention

The present application is based upon discovery of an improved method for identifying and isolating certain DNA sequences. This invention takes advantage of particular synthetic DNA probe sequences as hybridizing tools enabling such identification and isolation of the particular desired DNA sequences from a large heterogeneous gene population. Thus, the present invention provides a simple, and surprisingly successful, means for identifying and isolating a particular DNA sequence thus permitting its further study and use in subsequent recombinant DNA techniques which allows for its ultimate expression in a genetically altered host.

0383L

## Background of the Invention

Over the past years, the science of recombinant DNA technology has matured and enabled the preparation of a wide variety of heretofore substantially unobtainable proteins using certain genetically altered hosts. Examples of such now abound in the literature. In all these cases, the scientists involved were faced with the task of obtaining the prerequisite DNA sequence encoding the desired protein and inserting it by means of restriction sites into cloning and expression vehicles. When placed functionally in such vehicles, the DNA could be expressed so that, upon transcription and translation, an amino acid sequence corresponding to the desired protein is produced.

Generally, the most difficult step in the overall process of obtaining a desired protein by recombinant DNA technology has been to obtain the necessary gene for insertion in the expression vehicle, usually a plasmid for transformation of a microbial host. Initially, since the target proteins were relatively small, well characterized molecules, sophisticated organic chemical procedures could be used to synthesize the gene.

When the viability of the basic technology for expression of a heterologous gene in a suitable host had been demonstrated, more complicated proteins such as human growth hormone ( 190 amino acids), various interferons ( 166 amino acids), and human serum albumin were selected as targets. Often, however, only a portion of the amino acid sequence was known and chemical synthesis of the gene was, therefore, impossible. More importantly, the chemical synthesis of genes of the requisite size was not a practical process in any event. As a result, resort was had to means of isolating gene coding for more complex proteins from pools of complementary DNA (cDNA) produced in vitro for this purpose.

0383L

- 3 -

0128042

This procedure involved extracting messenger RNA from sources thought to be rich in messenger RNA coding for a particular protein. This involved the use of synthetic probe sequences known to be complementary to a portion of the desired messenger RNA transcript. By fashioning a unique short sequence complementary to a corresponding portion of the messenger RNA transcript it was possible, by radioactive labeling of the probe and hybridization techniques, to identify and isolate the corresponding cDNA.

This method necessarily involved the preparation of synthetic probes that were sufficiently unique so as to hybridize with the desired DNA sequence and no others. Logically, this compelled knowledge of the DNA sequence in order to fashion the properly encoded synthetic probe. This knowledge, in turn, demanded knowledge of the amino acid sequence of the desired underlying protein. Even armed with such knowledge, genetic code degeneracy, which allows base pair variation for a given amino acid choice frustrated attempts to efficiently and faithfully isolate the requisite DNA sequence.

In view of these difficulties, it was necessary to find stretches of amino acids which had minimal breadth of codon choice. In this manner, unique synthetic DNA probes complementary to the corresponding DNA stretch could be expected to bind the desired DNA in the genomic DNA bank. These considerations necessarily imposed restrictions on the research freedom of obtaining the requisite DNA sequences. Expenditures of much time and effort were necessary to examine the protein sequence so as to locate an appropriate subsequence having the limited variation required. Such restrictions provided incentives to identify new and improved methods by which the requisite DNA sequences could be identified and isolated from their sources.

In experiments embodying different approaches to solve this problem, it has been discovered that certain synthetic DNA probes

0383L

can be prepared, without regard to the degeneracy of the genetic code, which can be used successfully to hybridize with the desired DNA sequence. Such discovery is the subject of the present invention.

The publications and other materials hereof used to illuminate the invention, and in particular cases, to provide additional details respecting its practice are incorporated herein by this reference.

Summary of the Invention

The present invention, in its basic aspects, is directed to means and methods for the efficient and specific identification and isolation of a particular DNA sequence based upon use of synthetic DNA hybridization probe, fashioned irrespective of considerations for genetic code degeneracy. First, the present invention relates to a process for identifying and isolating a particular, desired DNA sequence from among a mixture of DNA, using as a hybridization probe, a synthetically produced DNA sequence of at least about 30 nucleotide base pairs, constructed without reference to any particular subsequence of the said desired DNA sequence, rather only to the corresponding amino acid sequence encoded by said desired DNA sequence. Second, the present invention relates to the synthetic DNA hybridization probes thus constructed as well as to the product of hybridization of it with the desired DNA sequences.

It will be understood that the present invention involves the use of a synthetic probe generally to identify and isolate a particular specific DNA sequence and is not to be limited to any particular desired embodiments. It has been found unnecessary to completely duplicate the nucleotide sequence of a gene fragment for a given protein to permit its successful use as a probe. Such probe can be constructed substantially without regard to or knowledge of the underlying DNA sequence nor to specific analysis of the amino acid sequence and its composition vis-a-vis variation in codon choice because of degeneracy in the genetic code. The basic requirements of this invention are only that the synthetic DNA hybridization probe accurately represent at least a portion of the complement to the desired DNA sequence with respect only to proper amino acid coding, and that it be of a length sufficient to ensure recognition and hybridization with the desired DNA sequence. The invention is otherwise practiced following hybridization techniques known per se.

Brief Description of the Drawings

Fig. 1 shows the homology between the nucleotide sequence of a portion of the naturally occurring gene for IGF-1 (Insulin-like Growth Factor) and that of a synthetic gene coding for the same amino acid sequence but having a different nucleotide sequence.

Fig. 2 illustrates plaque containing plates obtained in a first screen of plaques using a $^{32}$P-labeled synthetic IGF-1.

Fig. 3 illustrates a plaque containing plate obtained as a second screen of plaques taken from the region of the first screen giving a positive indication of hybridization with $^{32}$P-labeled IGF-1 probe.

Fig. 4 illustrates a Southern Blot Hybridization of rescreened plaques obtained using $^{32}$P-labeled IGF-1 DNA fragment as a probe.

Fig. 5 shows the homology between the nucleotide sequences of the genes for the A and B chains of human insulin and that of synthetic genes coding for the same amino acid sequences but having different nucleotide sequences.

0383L

Fig. 6 illustrates plaque containing plates obtained in a first screen of plaques using $^{32}$P-labeled synthetic probes for the A and B chains of insulin.

Fig. 7 illustrates a Southern Blot Hybridization of rescreened plaques obtained using $^{32}$P-labeled synthetic probes for the A and B chain of insulin.

Description of the Preferred Embodiments

The conditions necessary to practice the present invention involve the use of a synthetic hybridization probe, prepared considering only the amino acid sequence of the protein encoded by a desired DNA, ranging in length from about 30 base pairs upwards of one hundred of more. The nucleotide sequence of synthetic probe can be selectively hybridized, as desired, with a DNA sequence which corresponds to the coding or sense strand for the desired protein or its complement or negative sense strand, or both. In terms of overall homology between the DNA of the complement to the underlying, desired DNA sequence versus that of the hybridization probe, a range of from about 35 to about 60 percent or more, preferably at least 50 percent, is generally sufficient. Likewise, the length of the synthetic hybridization probe can range from about 30 to upwards of 100 base pairs, preferably from about 35 to 65 base pairs. As a general rule, however, the utilization of longer-probes and/or greater homology increases the specificity of hybridization with the particular desired DNA sequence, i.e., reduces the hybridization with undesired DNA sequences. In general, the conditions for successful hybridization are based upon utilizing a temperature from about 0° to about 50°C, preferably from about 30° to 45°C. A particular preferred temperature of about 42°C is satisfactory. Likewise, the hybridization is done in a salt solution of suitable concentration, for example, Denhardt's solution or any combination of other suitable salts. In some instances, an addition of up to 20 percent of formamide to the solution is useful.

0383L

Particularly if conditions of higher stringency are employed, it may be possible to have successful results with as few as about 20 base pairs in the probe, especially when homology is higher, or with probes having homology lower than 35 percent, especially where the probe length is at the high end of the range.

In addition, reference should be had to the detailed description which follows, giving further particular preferred conditions for practicing the present invention.

A. Synthesis of DNA Probes

A synthetic DNA sequence coding for a portion of the amino acid sequence of Insulin-like Growth Factor-1 (IGF-1), but not identical to the actual gene sequence, was obtained according to the following scheme.

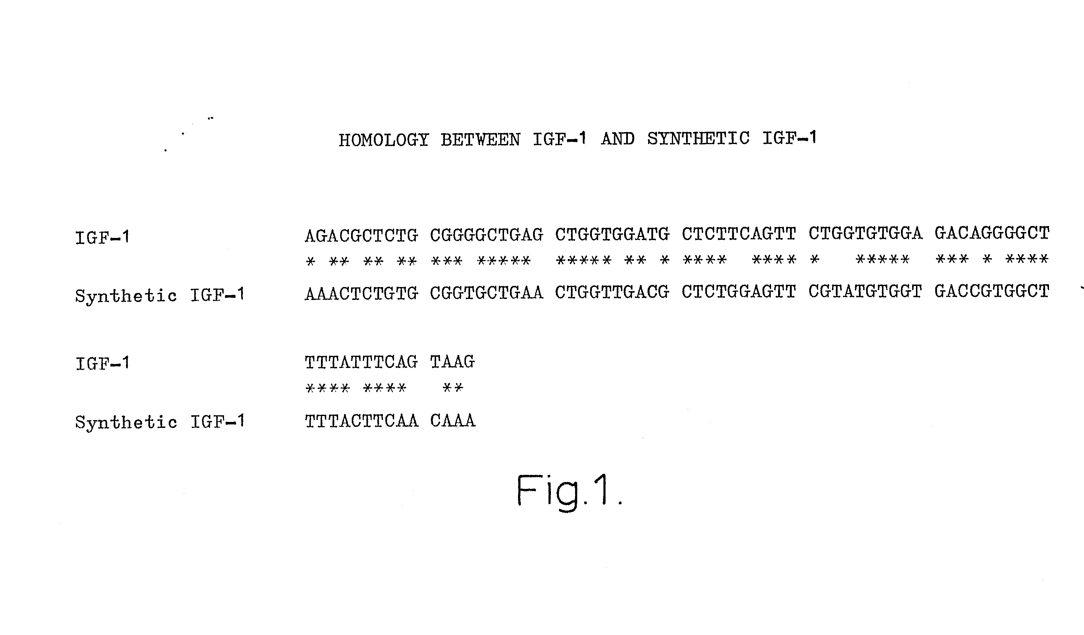

Eight fragments as shown in Fig. 1 were synthesized according to the method of Crea, R. and Horn. T., Nucleic Acids Research, 8: 2231 (1980), except that the condensing reagent used was mesitylenesulfonyl-3-nitrotriazole (MS-NT) (instead of 2,4,6-triisopropylbenzene sulfonyl tetrazole).

The synthesis of the fragments was accomplished from the appropriate solid support (cellulose) by sequential addition of the appropriate fully protected monomer-, dimer- or trimer- blocks. The cycles were carried out under the same conditons as described in the syntheis of oligothymidilic acid (see Crea et al., supra).

The final polymer (74 base pairs) was treated with base (aq. conc. $NH_3$) and acid (80 percent aq. HoAc), the polymer pelleted off and the supernatant evaporated to dryness. The residue, dissolved in 4 percent aq. $NH_3$, was washed with ether (3X) and used for the isolation of the fully deprotected fragment. Purification was accomplished on a 15 percent polyacrylamide gel and recovered by electroelution and ethanol precipitation.

0383L

The full length probe was constructed from the foregoing eight fragments using the general technique of Rossi, J. et al., J. Biochem., 257: 9226 (1982). The procedure involves construction of 4 double stranded sets using pairs of fragments chosen from these eight. Each pair is chosen so that there is a short region of complementary overlap. The overlapping pair is then treated with DNA polymerase I (Klenow) to create a double stranded blunt-ended extended sequence. The blunt ends are removed (optionally only some of them) by treating with suitable restriction enzymes, forming compatible restriction cleavage sticky ends. These permit subsequence control of ligation between the appropriate extended pair sequences.

To construct the IGF-1, the fragments were paired as follows (starting in order from the ultimate 5' end of the IGF-1): 1L with 3L; 2L with 4L; 1R with 3R; 2R with 4R. Each paired set was then treated with DNA polymerase I (Klenow) in the presence of a mixture of the four NTPs, under standard conditions.

The 1L-3L pair was double digested with EcoRI and PstI; the 2L-4L sequence with PstI and BamH; the 1R-3R segment with BAMHI and AvaII; and the 2R-4R pair with AvaII and Sal.

The upstream portion of the probe was then prepared by a three way ligation using the double digested 1L-3L and 2L-4L fragments along with pBR322 previously cleaved with EcoRI and BamHI, and cloning the ligation mixture into E.coli K12 strain 294 (ATCC No. 31446).

The downstream portion of the probe was constructed in somewhat more complex fashion. Each of the paired sequences was annealled into Pst cleaved pBR322 prior to the above described double digestion. The blunt ended fragment was dc tailed and annealled with dT tailed Pst cleaved pBR322, and transformed into E.coli. Plasmids were isolated from successful transformants and

0383L

sequenced to confirm correct construction. Plasmids isolated from clones containing the blunt ended 2L-4L pairs were double digested as described above with AvaII and Sal and the short fragment containing the desired double stranded Sal isolated by polyacrylamide gel and electroeluted. This fragment was then ligated with cleaved plasmids (with AvaII and Sal) derived from the clones containing the 1L-3L sequences. The resulting ligation mixture was cloned into E.coli, and transformants isolated to obtain plasmids containing the inserts coding for the 3' portion of the probe.

The vector fragment from the digestion of plasmids containing the 5' sequences of probe digested with BamHI and Sal was the ligated with the Bam-Sal insert containing the 3' sequences. The resulting plasmid contained the entire probe sequence of IGF-1 which could be excised and recovered by digestion with EcoRI and Sal, followed by purification of the desired short fragment using polyacrylamide gel and electroelution.

The homology between the synthesized sequence and the actual gene for IGF-1, 55 of 74 nucleotides, is shown in Fig. 1, homology being indicated by an asterisk.

B.  Preparation of Human Genomic Library

Human fetal liver DNA was prepared according to the procedure of Blin and Stafford, Nucleic Acid Research, 3: 2303 (1976) and was subject to a nonlimited digestion with the restriction endonucleases HaeIII and AluI, and the products were size-fractionated by sucrose gradient centrifugation. See Maniatis et al., Cell, 15: 1157-73 (1978). Large fragments (15-20 kb) were isolated and treated with EcoRI methylase to render EcoRI sites within the human DNA resistant to cleavage with EcoRI. Synthetic dodecameric DNa molecules bearing an EcoRI cleavage site (EcoRI linkers) were ligated to the methylated DNA and digested with EcoRI to generate EcoRI cohesive ends. Following an additional size

0383L

selection (15-20 kb), the human DNA was suitable for insertion into the bacteriophage cloning vector, Charon 4A (λCh4A).

Foreign DNA can be inserted into the λCh4A vector after the removal of two internal EcoRI fragments which contain genes nonessential for phase growth. The two "arms" of the bacteriophage DNA are annealled through their 12 base pair cohesive ends and joined to the human DNA by ligation of the EcoRI cohesive ends. The ligation reaction is performed at a high DNA concentration to promote the formation of long concatemeric DNA molecules which are the substrate for in vitro packaging. Approximately 1 x $10^6$ in vitro packaged phage were amplified $10^6$ fold by low density growth on agar plates to establish a permanent library of cloned human DNA fragments.

C.  Screening the Genomic Library

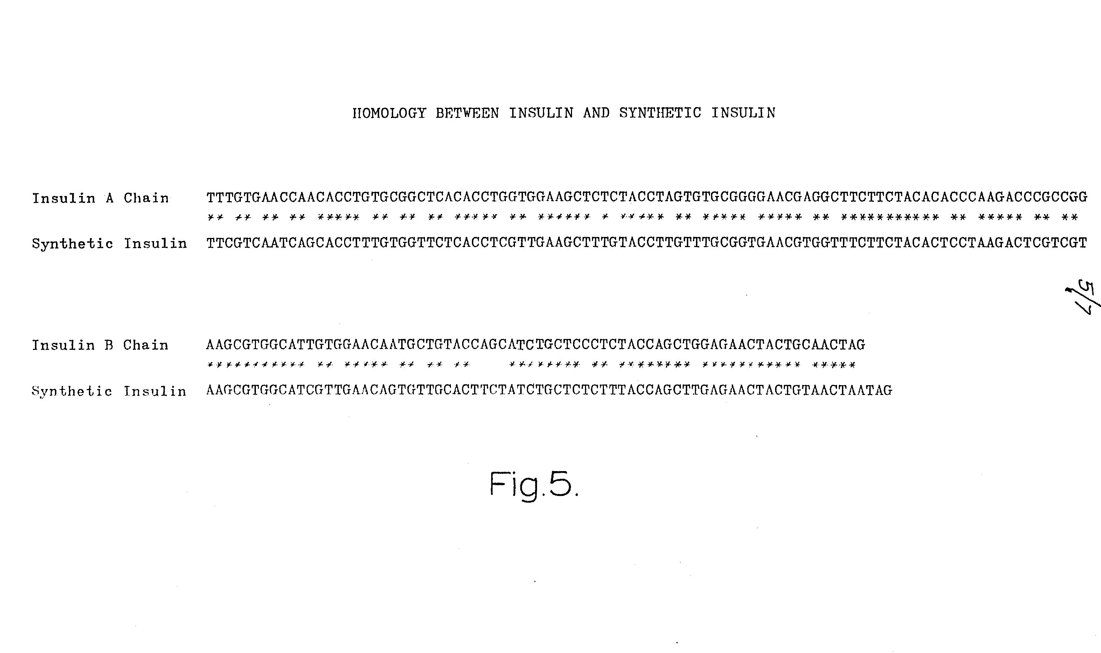

The HaeIII-Alu library made from human fetal liver was screened using the in situ plaque hybridization technique of Benton and Davis, Science, 196: 180 (1977). 100,000 recombinant phage were plated on 3.1 x $10^8$ exponential phase bacterial cells on 15 cm NZCYM petri dishes. To prevent top agar from adhering to the nitrocellulose filter when it was lifted from the plate (which tends to increase the background hybridization), plates were dried in a 37 C incubator for several hours or set on edge overnight to drain excess liquid. The use of 0.7 percent agarose rather than agar in the top agar layer also minimized this problem. The plates were incubated at 37 C for 14-16 hrs., at which time the plaques were confluent. Plates were refrigerated for an hour or longer before the filters were applied. Nitrocellulose filters (pore size 0.45 m) fit easily over the agar plate. Phage and DNA were adsorbed to these filters in duplicate, 1a and 1b, 2a and 2b, etc., by placing two filters on each plate sequentially, 5 min for each, at room temperature. Small holes were made with a needle filled with ink to orient the filters on the plates (see Fig. 5). The DNA was denatured and bound to the filters as described by Benton and Davis, supra.

0383L

To prepare the filters for hybridization to a labeled synthetic probe, they were wetted in about 10 ml per filter of 5X SET, 5X Denhardt's solution (5X Denhardt's solution = 0.1 percent bovine serum albumin, 0.1 percent polyvinylpyrolidone, 0.1 percent Ficoll; Denhardt, Biochem. Biophys. Res. Commun., 23: 641 (1966), 50 g/ml denatured salmon sperm DNA, and 0.1 percent sodium pyrophosphate + 2 percent formamide. The filters were prehybridized with continuous agitation at 42 for 14-16 hrs. The filters were hybridized with agitation at 42 in prehybridization solution containing a $^{32}$P-labeled hybridization probe. The synthetic probes for IGF-1, Insulin A chain and Insulin B chain were labeled using the procedure of Taylor et al., Biochem. Biophys. Acta, 442: 324-330 (1976) (see Fig. 1). After hybridization, the filters were washed 6X with agitation in about 15 ml per filter of 0.1 percent SDS, 0.1 percent sodium pyrophosphate, 0.2X SET at 37 C for 45 min. The filters were blotted dry, mounted on cardboard and exposed to Kodak XR5 X-ray film with Dupont Cronex 11R Xtra Life Lightning-plus intensifying screens at -70 C for 1-2 days.

D. Plaque Purification of Recombinant Phage

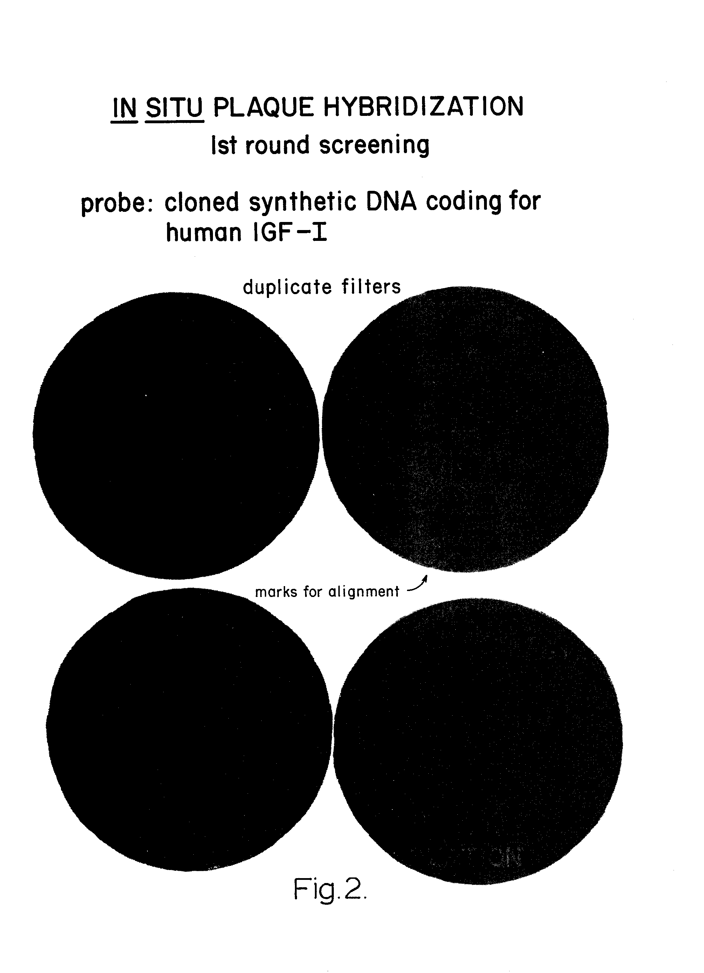

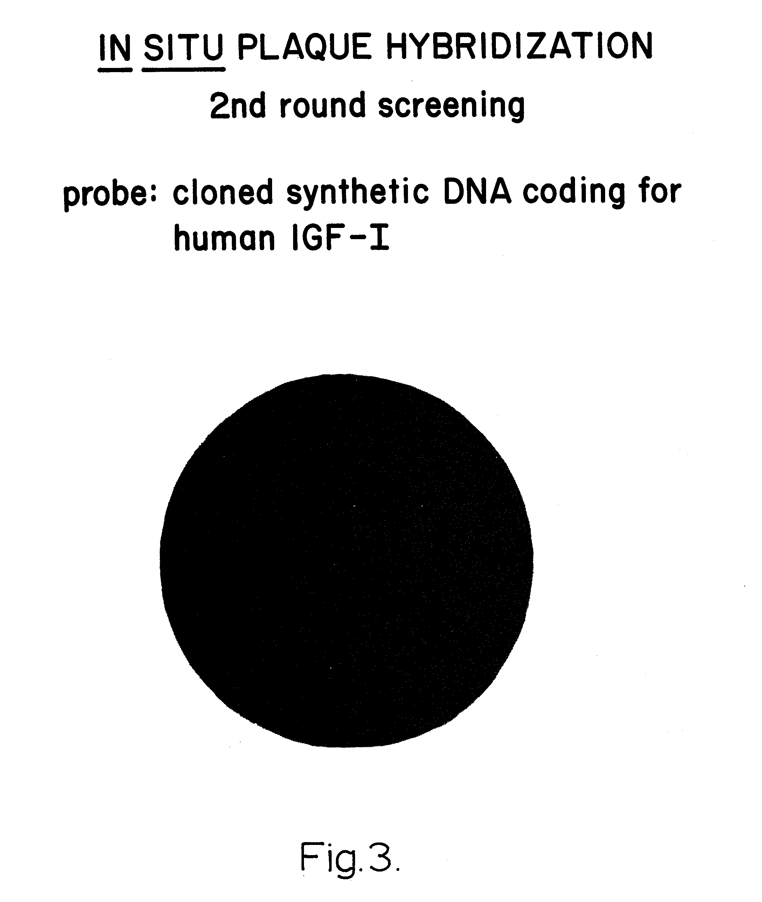

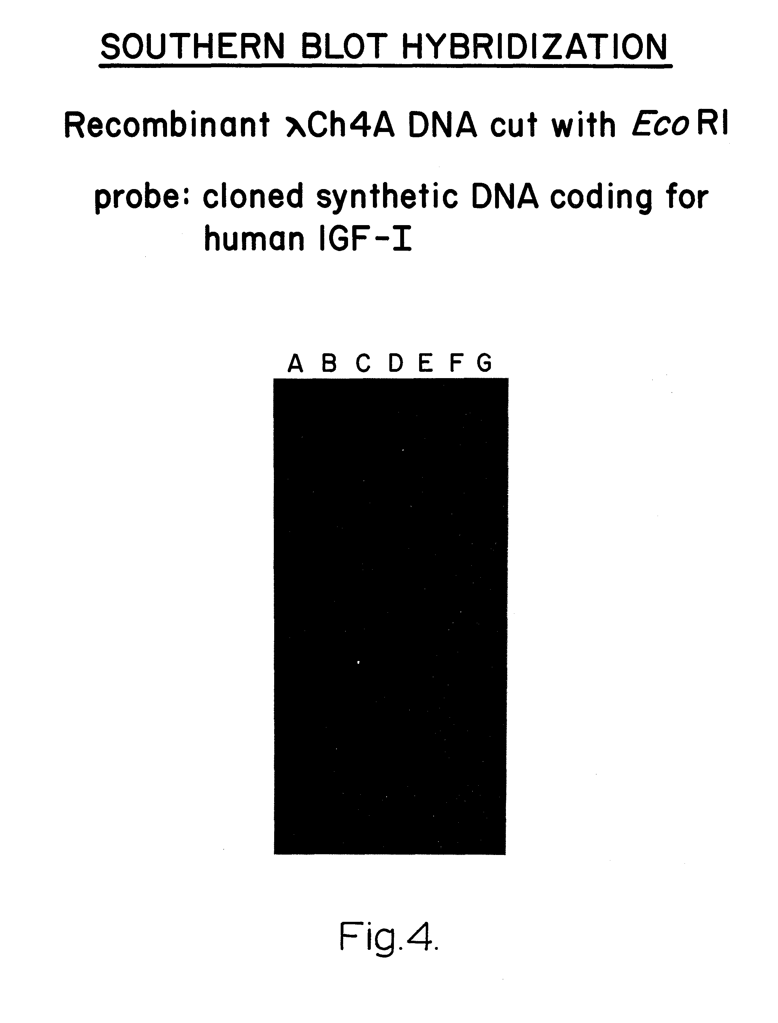

Plaques from the region of a plate corresponding to a positive on the autoradiogram for IGF-1 were picked and suspended in 0.5 ml PSB (see Fig. 2). The phage suspension was titered and the plate containing about 1,000 plaques was rescreened. The process of picking positives and rescreening was repeated until 90 percent of the plaques on a plate gave positive signals after screening (see Fig. 3). Single phage were suspended in 1.0 ml PSB (0.05 percent gelatin, 0.10 M NaCl, 0.01 M Tris pH 7.4, 0.01 M MgCl$_2$) at 37 C for 2 hrs. 50 was added to 0.2 ml of exponential phase bacterial cells + 0.2 ml of 10 mM MgCl$_2$, 10 mM CaCl$_2$, 37 /15". This was added to 50 mls NZYDT mediat 37 /14-16 hrs. Chloroform + 3g NaCl was added and the bacteria removed by pelleting 15"/5K. 3.5g of polyethylene glycol was added to the supernatant/1 hr. 0 . The phage precipitated and were pelleted by spinning 20"/5K. The pellet

0383L

was resuspended in 2.0 mls PSB + 1.0g CsCl and layered on 0.9 ml steps of 1.7, 1.5 and 1.45g/cc CsCl in PSB. The gradient was spun at 25K in a Sorval 50 Ti for 3 hrs. The phage band was collected and dialyzed 14-16 hrs. against 0.1M NaCl, 50 mM Tris-Cl (pH 7.5) 10 mM MgSO$_4$. The phage DNA was extracted with phenol equilibrated with 10 mM Tris, 1 mM EDTA. The phenol was removed with chloroform and the DNA was precipitated with 100 percent ethanol. The DNA was resuspended in water and restricted with EcoRI at 37 . The digested DNA was run on a 1 percent agarose gel. Blot hybridization analysis of DNA digests was carried out according to Southern, J. Mol Biol. 98: 503-517 (1975). These filters were probed under the same conditions as the phage library. All seven of the phage DNAs, A, B, C, D, E, F, G, hybridized with the synthetic IGF-1 probe (see Fig. 4). Five of the seven had identical U.V. patterns. The 3.0 kb RI fragment that hybridized was restriced with Sau3A and cloned into M13 (ATCC 15669-B1). Plaque lifts were done as previously described and DNA was made from the hybridizing plaques. The DNA sequences when translated gave the same amino acid pattern as IGF-1 thereby confirming that it was IGF-1. Subsequently this fragment that codes for the IGF-1 coding sequence was labeled as previously described and used as a probe on all 7 of the restricted phage DNAs. The conditions for hybridization were the same as previously described except the percentage of formamide was increased to 50 percent, making these conditions more stringent. The washes were now done at 55 . All 7 of the DNAs hybridized, confirming that all 7 plaques originally identified using a synthetic IGF-1 probe were indeed IGF-1.

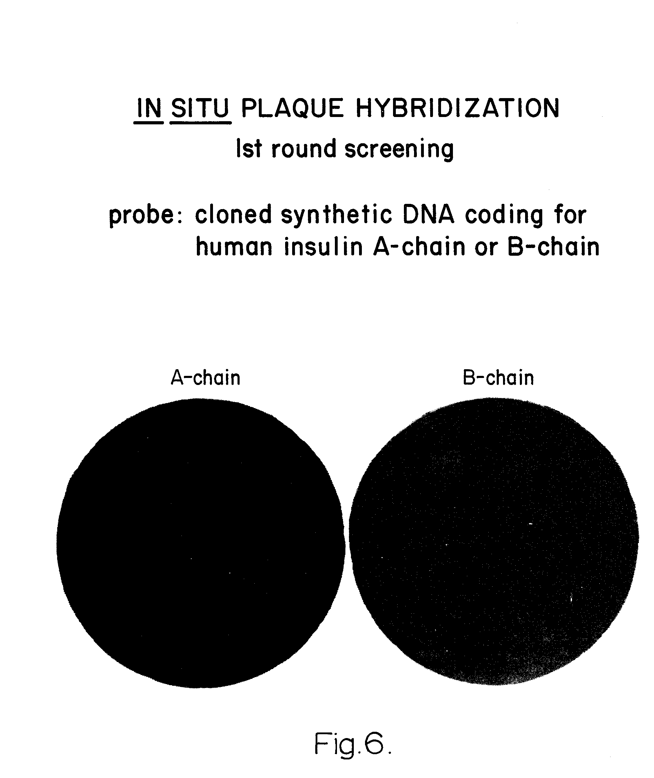

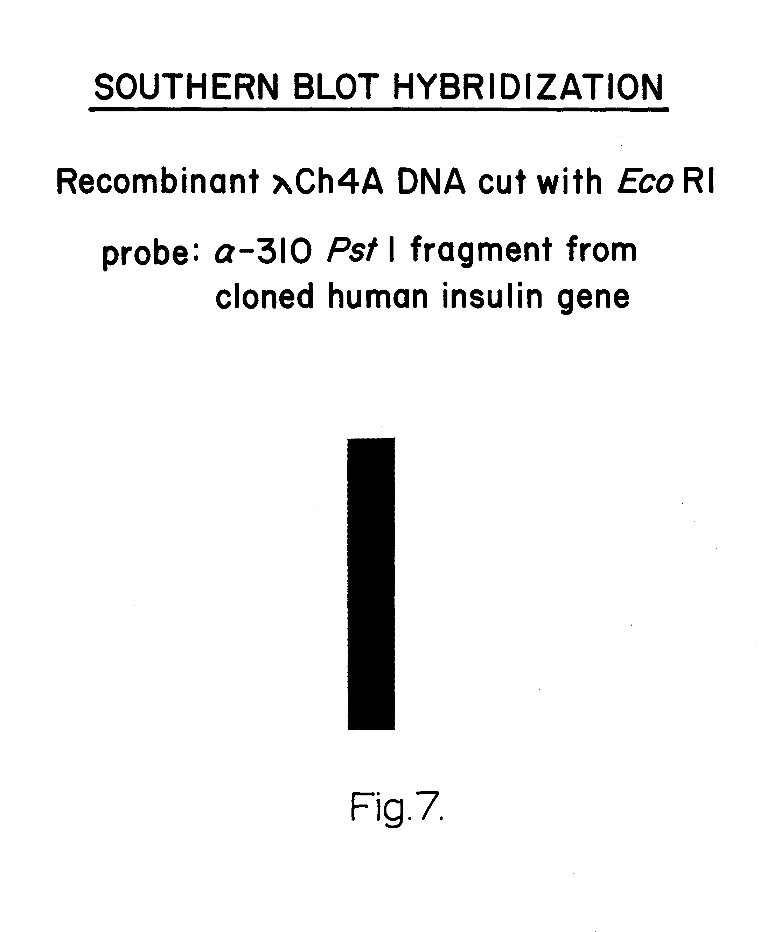

To demonstrate that the use of a single synthetic DNA probe to identify a gene had general applications, the human library was further screened fo the insulin gene using the two synthetic A and B insulin probes of Fig. 5. The probes are from two different regions of the gene for insulin corresponding to its gene for the A and B chain. A plaque that hybridized with both probes was picked (see Fig. 6). Rescreening was repeated and phage DNA was made as

0383L

described above in connection with IGF-1. Blot hybridization of DNA digest was carried out according to Southern, supra (see Fig. 7). The phase DNA identified with the synthetic insulin probes codes for the human insulin gene 310 bp Pst fragment that has been sequenced and is known to code for human insulin. See Nucleic Acids Research 10: 2225-2240 (1982) and Science 209: 612 (1980).

From the foregoing it can be seen that synthetic DNA probes made and used in accordance with the present invention can be used to identify and isolate a gene coding for desired protein which is contained in a gene bank comprising a complex mixture of DNA fragments. Therefore, notwithstanding that the invention has been illustrated by reference to specific embodiments, those skilled in the art will recognize its wider applicability. Accordingly, the invention is to be construed as limited only by the appended claims.

0383L

-14-

CLAIMS

1. A process for identifying or isolating a DNA sequence which codes for a desired protein or portion thereof or a DNA sequence complementary to said coding sequence comprising hybridizing the desired DNA sequence with a synthetic DNA hybridization probe which probe comprises at least about 30 nucleotide base pairs in a sequence which codes for a portion of the protein, the nucleotides having been selected without regard to the degeneracy of the genetic code but wherein the homology between the synthetic probe and the actual DNA sequence in the gene for the desired protein is at least about 35 percent.

2. A process according to Claim 1 wherein the synthetic probe comprises about 30 to about 100 base pairs.

3. A process according to Claim 2 wherein the synthetic probe is from about 35 to 60 base pairs.

4. A DNA hybridization probe synthetically prepared having at least about 30 nucleotide base pairs and at least about 35 percent homology with an actual DNA gene sequence encoding a desired protein or portion thereof.

5. A DNA probe of Claim 4 hybridized with an actual DNA gene sequence encoding a desired protein or portion thereof.

6. The probe according to Claim 4 or 5 useful for hybridizing with the DNA gene sequence encoding IGF-1.

7. The probe according to Claim 6 having the sequence as depicted in Fig. 1 hereof.

0383L

HOMOLOGY BETWEEN IGF-1 AND SYNTHETIC IGF-1

IGF-1

AGACGCTCTG CGGGGCTGAG CTGGTGGATG CTCTTCAGTT CTGGTGTGGA GACAGGGGCT

\* \*\* \*\* \*\* \*\*\* \*\*\*\*\* \*\*\*\*\* \*\* \* \*\*\*\* \*\*\*\* \* \*\*\*\*\* \*\*\* \* \*\*\*\*

Synthetic IGF-1

AAACTCTGTG CGGTGCTGAA CTGGTTGACG CTCTGGAGTT CGTATGTGGT GACCGTGGCT

IGF-1

TTTATTTCAG TAAG

\*\*\*\* \*\*\*\* \*\*

Synthetic IGF-1

TTTACTTCAA CAAA

Fig.1.

# IN SITU PLAQUE HYBRIDIZATION
## Ist round screening

probe: cloned synthetic DNA coding for
human IGF−I

duplicate filters

marks for alignment

Fig.2.

# IN SITU PLAQUE HYBRIDIZATION
## 2nd round screening

## probe: cloned synthetic DNA coding for human IGF-I

Fig.3.

## SOUTHERN BLOT HYBRIDIZATION

## Recombinant λCh4A DNA cut with *Eco* RI

## probe: cloned synthetic DNA coding for human IGF-I

A B C D E F G

Fig.4.

HOMOLOGY BETWEEN INSULIN AND SYNTHETIC INSULIN


Insulin A Chain    TTTGTGAACCAACACCTGTGCGGCTCACACCTGGTGGAAGCTCTCTACCTAGTGTGCGGGGAACGAGGCTTCTTCTACACACCCAAGACCCGCCGG

                   ** ** ** ** ***** ** ** ** ***** ** ****** * ***** ** ***** ***** ** ********** ** ***** ** **

Synthetic Insulin  TTCGTCAATCAGCACCTTTGTGGTTCTCACCTCGTTGAAGCTTTGTACCTTGTTTGCGGTGAACGTGGTTTCTTCTACACTCCTAAGACTCGTCGT


Insulin B Chain    AAGCGTGGCATTGTGGAACAATGCTGTACCAGCATCTGCTCCCTCTACCAGCTGGAGAACTACTGCAACTAG

                   ********** ** ***** ** ** **       ******* ** ******** ********** *****

Synthetic Insulin  AAGCGTGGCATCGTTGAACAGTGTTGCACTTCTATCTGCTCTCTTTACCAGCTTGAGAACTACTGTAACTAATAG


# Fig.5.

## IN SITU PLAQUE HYBRIDIZATION
### Ist round screening

probe: cloned synthetic DNA coding for
human insulin A-chain or B-chain

A-chain                              B-chain

Fig.6.

# SOUTHERN BLOT HYBRIDIZATION

Recombinant λCh4A DNA cut with *Eco* RI

probe: *a*-310 *Pst* I fragment from
cloned human insulin gene

Fig. 7.